# EUROPEAN PATENT APPLICATION

(11) **EP 4 286 443 A1**
(43) Date of publication of application: **06.12.2023**
(21) Application number: 22460050.2
(22) Date of filing: 20.10.2022
(51) Int. Cl.: C08G 61/12, C08L 65/00, C09D 165/00, H10K 30/00

(54) **NEW PERINONE POLYMER AND METHOD OBTAINING A PERINONE POLYMER IN THE PROCESS OF POTENTIOSTATIC POLYMERIZATION**

(30) Priority: 03.06.2022 PL 44136922
(71) Applicant: Politechnika Slaska, 44-100 Gliwice (PL)
(72) Inventor: Lapkowski, Mieczyslaw, 44-207 Rybnik (PL); Janasik, Patryk, Ozesze (PL); Czichy, Malgorzata, 40-404 Katowice (PL)

(57) **Abstract**

A new perinone polymer with the general formula containing units marked as u, w, v, x, y, z: 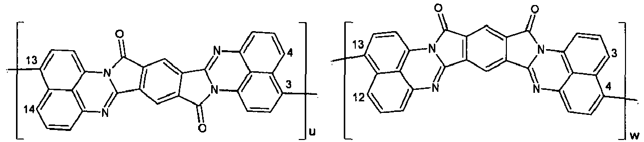 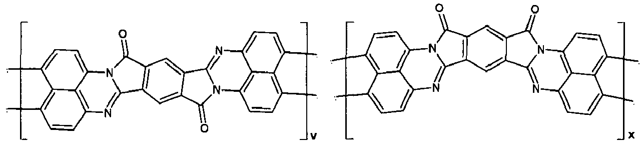 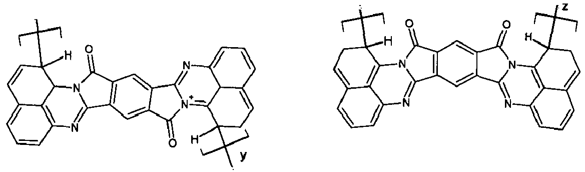 where:
u - poly(*anti*M) by single bond via positions 1, 2, 3, 4, 5, 6, 11, 12, 13, 14, 15 and/or 16
w - poly(*syn*M) by single bond via positions 1, 2, 3, 4, 5, 6, 10, 11, 12, 13, 14 and/or 15
v - poly(*anti*M) by double bond via positions 1, 2, 3, 4, 5, 6, 11, 12, 13, 14, 15 and/or 16
x - poly(*syn*M) by double bond via positions 1, 2, 3, 4, 5, 6, 10, 11, 12, 13, and/or 15 y - poly(*anti*M) by unprotonated bond via positions 1, 2, 3, 4, 5, 6, 11, 12, 13, 14, 15 and/or 16
z - poly(*syn*M) by unprotonated bond via positions 1, 2, 3, 4, 5, 6, 10, 11, 12, 13, 14 and/or 15.

The invention also relates to a method of obtaining a perinone polymer in the process of potentiodynamic polymerization.

## Description

The subject of the patent is a new perinone polymer and a method of obtaining a perinone polymer in the process of potentiodynamic polymerization.

Π-conjugated polymers are used extensively due to the simplicity with tailoring of their electric conductive properties. Most π-conjugated polymers have low electrical conductivity or are electrical insulators in their neutral state, e.g. poly (p-phenylene). Conductivity can be increased by some physical agents - electromagnetic radiation (photoconductivity); heating (thermoconductivity); as well as chemical modification - oxidation processes (p-doping), e.g. polythiophene and their derivatives (Dengke Zhaoa, Ligui Li, Wenlian Niua, Shaowei Chena, Highly conductive polythiophene films doped with chloroauric acid for dual-mode sensing of volatile organic amines and thiols, Sensors and Actuators B: Chemical 2017, 243, 380-387, doi: 10.1016/j.snb.2016.12.018); reduction process (n-doping), e.g. stable cations and dications in polymeric segments - polyvinylpyrrolidone and their derivatives. Developing the π-conjugated polymer with high electronic conductivity in a neutral state continues to be the key challenge. Increasing the electronic conductivity of π-conjugated polymers can be realized by increasing the effective conjugation length of the main chain, maximizing the effects of side substituents, increasing long-range interactions, and obtaining donor-acceptor, condensed or partially condensed (ladder or partially ladder polymers) structures (CLP- ang. *Conjugated Ladder Polymers*). In particular, the last structural approach appears to be of interest, where conjugated two-dimensional structures were obtained by intramolecular fusing aromatic or heteroaromatic precursors intramolecularly by an oxidation process. The fused two-dimensional structure limits the torsional rotation between aromatic units along the conjugated backbone. Among these partially or completely ladder polymers are polyquinoxalines (P.M. Beaujuge, C.M. Amb, J.R. Reynolds, Spectral engineering in π-conjugated polymers with intramolecular donor-acceptor interactions, Acc. Chem. Res. 43 (2010) 1396-1407. https://doi.org/10.1021/ar100043u), poly(phenazine) (M. D. Pace, O. K. Kim, (1988). Conducting properties and electron paramagnetic resonance of polyphenothiazine and polyphenoxazine ladder polymers, Synth. Met, 1988, 25, 333-339. doi: 10.1016 / 0379-6779 (88) 90573-5); poly(phenoxazines) and carbazole derivatives. In turn, perinones are the carbonyl class of pigments made from naphthalenetetracarboxylic dianhydride and o-phenylenediamine, while their most popular CLP derivative is ladder poly(benzobisimidazobenzophenanthroline) (BBL). Additionally, several findings in the literature mention the reactivity of other perinone monomers obtained by condensation from 1,8-naphthalenediamines (Czichy, M.; Motyka, R.; Zassowski, P.; Grabiec, E.; Janasik, P.; Brzeczek-Szafran, A.; Laba, K.; Wolinska-Grabczyk, A.; Lapkowski, M. Effects of solution-phase ordering on the spectroscopic properties and electrooxidative reactivity of isomeric mixtures and isolated isomers of synthesized amidine derivatives. Dye Pigment. 2020, 178, 108309 and Czichy, M.; Janasik, P.; Motyka, R.; Zassowski, P.; Grabiec, E.; Wolinska-Grabczyk, A.; Lapkowski, M. Influence of isomeric phthaloperinone monomers on the formation of π-dimers and σ-bonded segments in electrochemically-crosslinked products. Electrochim. Acta 2021, 370, 137669). For this reason, we have proposed perinones with terminal perimidine groups as novel monomers for ladder polymers.

The invention relates to a perinone polimer, which is characterized by electroactivity, bipolarity, n-type conductivity and a low energy gap. This polymer can be used in the production of OLEDs, NIR diodes, (bio)sensors, memory devices, alternatives for metallic conductors, anti-corrosion coatings, etc. (R. L. Van Deusen, J. Polym. Sci., Part B: Polym. Lett., 1966, 4, 211-214).

The aim of the invention is to develop a perinone polymer and a method of its preparation, which will allow to obtain a material with a high n-type conductivity and a low energy gap.

The content of this invention is a new perinone polymer, and its general formula contains units marked as u, w, v, x, y, z: 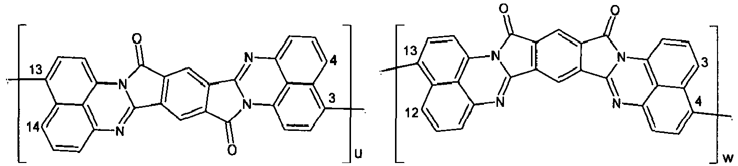 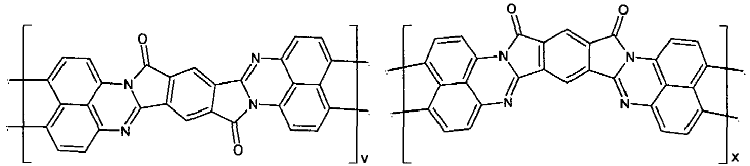 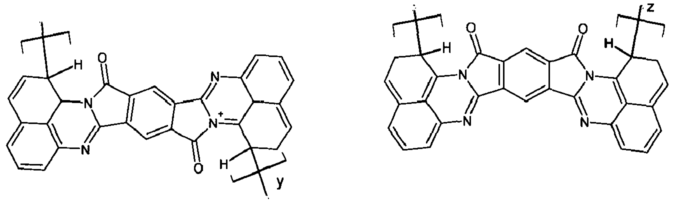 where:
u - poly(*anti*M) by single bond via positions 1, 2, 3, 4, 5, 6, 11, 12, 13, 14, 15 and/or 16
w - poly(*syn*M) by single bond via positions 1, 2, 3, 4, 5, 6, 10, 11, 12, 13, 14 and/or 15
v - poly(*anti*M) by double bond via positions 1, 2, 3, 4, 5, 6, 11, 12, 13, 14, 15 and/or 16
x - poly(*syn*M) by double bond via positions 1, 2, 3, 4, 5, 6, 10, 11, 12, 13, and/or 15 y - poly(*anti*M) by unprotonated bond via positions 1, 2, 3, 4, 5, 6, 11, 12, 13, 14, 15 and/or 16
z - poly(*syn*M) by unprotonated bond via positions 1, 2, 3, 4, 5, 6, 10, 11, 12, 13, 14 and/or 15

The content of the invention is also a method of obtaining a perinone polymer in the process of potentiodynamic polymerization in the mixture of *anti*M+*syn*M (*anti* perimidino[1',2':1,5] pyrrolo[3,4-*m*]phthaloperine-9,19-dione and *syn* 17H, 19H-perimidino[1',2':1,2]pyrrolo[3,4-*m*]phthaloperine-17,19-dione in a molar ratio from 1:0 to 0:1), using cyclic voltammetry on a working electrode made of metallic or carbon materials; preferably platinum, a platinum alloy or a glassy carbon, where the process is carried out under the conditions of:
- recommended sweeping potential between 0.5 V and 1 V against the redox pair of ferrocene/ ferrocenium, reaching the oxidation peak of *anti*M+*syn*M (anodic mode),
- recommended sweeping potential between -1.75 V and 1 V against the redox pair ferrocene/ferrocenium, reaching the oxidation and reduction peak of *anti*M+*syn*M (anodic-cathodic mode),
- potential sweep, preferably in the range of 10 - 500 mV/s,
- deposition from the solution *anti*M+*syn*M with solvent as toluene or benzene and electrolyte as tetrabutylammonium tetrafluoroborate, tetrabutylammonium hexafluorophosphate or tetraammonium hexafluorophosphate,
- deposition from solid state of *anti*M+*syn*M in solvent as dichloromethane, chlorobenzene, 1,2-dichlorobenzene or methanol with electrolyte as form of tetrabutylammonium tetrafluoroborate, tetrabutylammonium hexafluorophosphate, tetraethylammonium hexafluorophosphate,
   the process is carried out until the PPPI product is deposited on the working electrode.

### Example I.

The perinone polymer with a poly(perimidinopyrrolo[3,4-*f*]isoindole) skeleton (PPPI) was obtained by electropolymerization from a mixture of perimidino [1',2':1,5]pyrrolo[3,4-*m*]phthaloperine-9,19-dione and 17H,19H-perimidino[1',2':1,2]pyrrolo[3,4-*m*]phthaloperine-17,19-dione. The PPPI polymer is electrically conductive, has complex redox activity, mixed conductivity and a low bad gap.

### Materials

The following materials were used: pyromellitic anhydride (1), 1,8-naphthalenediamine (2), tetrabutylammonium tetrafluoroborate, dichloromethane, toluene, benzene, chlorobenzene, 1,2-dichlorobenzene, nitrobenzene, methanol and isobutanol.

### Synthesis of monomers antiM+synM

Mixture of monomers (*anti*M+synM) as a mixture of *anti* isomer perimidino[1',2':1,5]pyrrolo[3,4-*m*] phthaloperine-9,19-dione and *syn* isomer 17H,19H-perimidino[1',2':1,2]pyrrolo[3,4-*m*] phthaloperine-17,19-dione) was obtained by condensation reaction of 1 compound (0.218 g, 1 mmol) with 2 compound (0.316 g, 2 mmols) (FIG. 1). Compound 2 was purified by recrystallization in methanol before use. The reaction was carried out in 5 ml of nitrobenzene for 3h under reflux. After finished reaction, the mixture was cooled to room temperature. The precipitate was filtered off and then washed with methanol. Then the precipitate was heated in toluene for 1h, and then in methanol for 1h under reflux. The precipitate was filtered off and dried in a vacuum. *Anti*M+*syn*M was obtained as a purple powder (0,273 g, 59%) Tₘ: >450 °C. ¹ H-NMR: δ/ppm (300 MHz, CDCl₃ with the addition of 10%-CF₃COOD, Me₄Si, mixture of *anti* and *syn* isomers) = 11.02 (CF₃COOH); 9.26 (*syn*, s, 1H); 9.1 (*anti*, s, 2H); 8.87 (*syn*, s, 1H); 8.58 (*anti*, d, 2H, J = 7,7 Hz; *syn*, d, 2H, J = 7.7 Hz); 7.78 (*anti*, d, 2H, J = 7.9 Hz; *syn*, d, 2H, J = 7.9 Hz); 7,73 (*anti*, d, 2H, J = 8.3 Hz; *syn*, d, 2H, J = 8.3 Hz); 7,64-7,55 (*anti*, m, 6H; *syn*, m, 6H). IR: *v*ₘₐₓ/cm⁻¹ = 1714, 1645, 1576, 1498, 1482, 1456, 1403, 1385, 1363, 1329, 1278, 1222,1206, 1166, 1145, 1133, 1105, 1032, 993, 885, 827, 772, 749, 702.

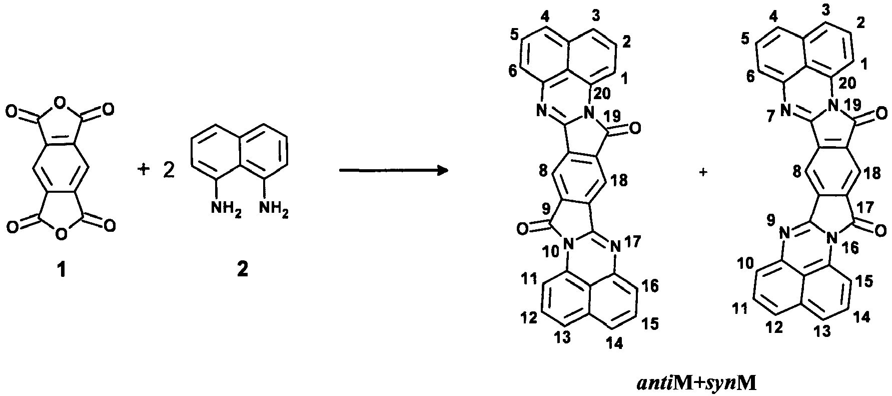

FIG. 1. Diagram showing the synthesis of *anti*M+*syn*M monomer mixture for the preparation of the PPPI polymer.

### Preparation of PPPI polymer

PPPI is obtained under potentiodynamic polymerization (cyclic voltammetry) in a solution of *anti*M+*syn*M and an electrolyte, preferably tetrabutylammonium tetrafluoroborate in dichloromethane, chlorobenzene or 1,2-dichlorobenzene. Potentiodynamic synthesis of PPPI can be performed in the anodic mode (FIG. 2a) or in the anodic-cathodic mode (FIG. 2b). Obtaining of PPPI in anodic mode must provide a potential sweep with a range of potentials including the oxidation peak of *anti*M+*syn*M*.* Obtaining of PPPI in the anode-cathode mode must provide a potential sweep within the potential limits of the oxidation and the reduction peak of *anti*M+*syn*M*.* The onset of the oxidation peak of *anti*M+*syn*M begins above 0.50 V, versus the redox pair of ferrocene/ferrocenium couple. The first reduction peak of *anti*M+*syn*M begins below -0.70 V, versus redox pair of ferrocene/ferrocene couple. The PPPI polymer can be deposited at different potential sweep rates on a various metallic, carbon, organic and inorganic surfaces that are electrically conductive. The polymerization can also beperformed in the solid state using the potentiodynamic or potentiostatic method.

PPPI polymer is characterized by wide charging currents in the potential range from - 2 V to 1.7 V, with reversible redox pairs at 0.1/-0.15 V, 0.45/0.40 V, -1.35/-1.55 and -1.85/-2.00 V against the redox ferrocene/ferrocenium pair. The levels of the HOMO, LUMO and LUMO+1 orbitals were calculated for the *anti*M+*syn*M precursors and alternative products, and thus the poly(perimidinopyrrolo[3,4-*f*] isoindole) skeleton structure was found in the PPPI product, as shown in FIG. 3.

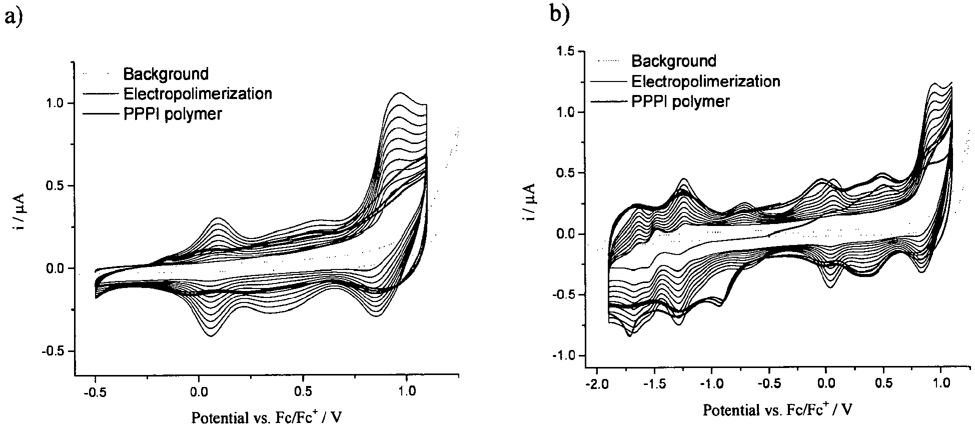

FIG. 2. Potentiodynamic method: cyclic voltammograms of the PPPI polymerization process in anodic (a) and anodic-cathodic (b) modes.

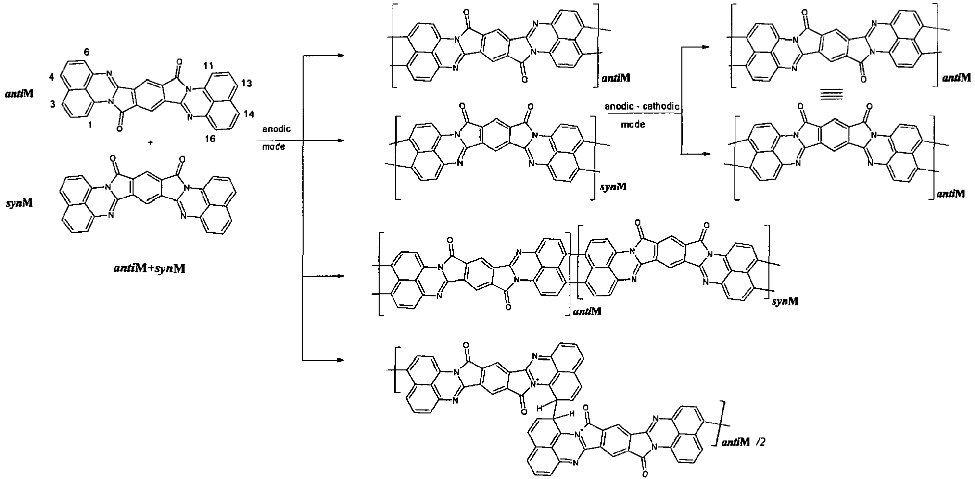

FIG. 3 Diagram of the mechanism of electrochemical preparation of PPPI and possible structure of PPPI, obtained in anodic and anodic-cathodic modes.

### Method for PPPI obtaining

A mixture of *anti*M+*syn*M monomers (*anti* perimidino[1',2':1,5]pyrrolo[3,4-*m*]phthaloperine-9,19-dione and *syn* 17H,19H-perimidino[1',2':1,2]pyrrolo[3,4-*m*] phthaloperine-17,19-dione, preferably in a molar ratio in the range of 1:0 to 0:1, is subjected to potentiodynamic polymerization (cyclic voltammetry) under the following conditions:
- sweeping potential between 0.5 V and 1 V against the redox pair of ferrocene/ferrocenium, reaching the oxidation peak of *anti*M+*syn*M (anodic mode),
- sweeping potential between -1.75 V and 1 V against the redox pair ferrocene/ferrocenium, reaching the oxidation and reduction peak of *anti*M+*syn*M (anodic-cathodic mode),
- speed rate of the potential in the range of 10 - 500 mV/s,
- material of the working electrode - metallic or made of carbon materials, e.g. platinum, platinum alloys, glassy carbon etc.,
- deposition from the solution of *anti*M+*syn*M, solvents, preferably toluene, benzene, and an electrolyte that is dissolved in a solvent, preferably tetrabutylammonium tetrafluoroborate, tetrabutylammonium hexafluorophosphate or tetraethylammonium hexafluorophosphate,
- deposition from solid state of *anti*M+*syn*M, solvents, e.g. dichloromethane, chlorobenzene, 1,2-dichlorobenzene, methanol and electrolyte which is dissolved in a solvent, e.g. tetrabutylammonium tetrafluoroborate, tetrabutylammonium hexafluorophosphate, tetraethylammonium hexafluorophosphate,
- time of the process -up to the precipitation of the PPPI product on the working electrode.

As a result of the method, depending on the mode type (anodic or anodic-cathodic mode), the following perinone polymer was formed with the formula: 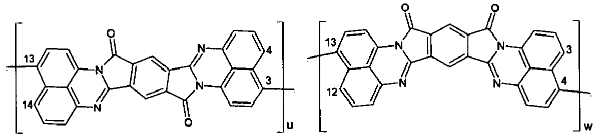 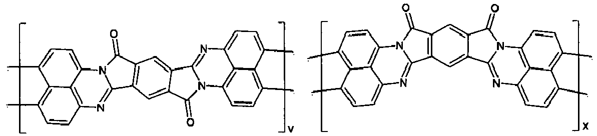 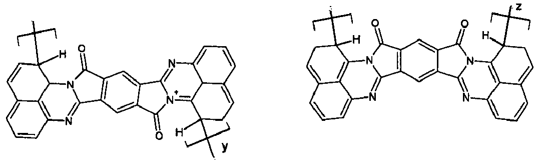 where:
u - poly(*anti*M) by single bond via positions 1, 2, 3, 4, 5, 6, 11, 12, 13, 14, 15 and/or 16
w - poly(*syn*M) by single bond via positions 1, 2, 3, 4, 5, 6, 10, 11, 12, 13, 14 and/or 15
v - poly(*anti*M) by double bond via positions 1, 2, 3, 4, 5, 6, 11, 12, 13, 14, 15 and/or 16
x - poly(*syn*M) by double bond via positions 1, 2, 3, 4, 5, 6, 10, 11, 12, 13, and/or 15
y - poly(*anti*M) by unprotonated bond via positions 1, 2, 3, 4, 5, 6, 11, 12, 13, 14, 15 and/or 16
z - poly(*syn*M) by unprotonated bond via positions 1, 2, 3, 4, 5, 6, 10, 11, 12, 13, 14 and/or 15

### Example II

Example II differs from the I in that the PPPI is obtained using the following procedure.

A mixture of *anti*M+*syn*M monomers (*anti* perimidino [1',2':1,5]pyrrolo[3,4-*m*]phthaloperine-9,19-dione and *syn* 17H,19H-perimidino[1',2':1,2]pyrrolo[3,4-*m*]phthaloperine-17,19-dione, preferably in a molar ratio of 1:0, is subjected to potentiodynamic polymerization (cyclic voltammetry) under the conditions:
- sweeping potential between 0.5 V and 1 V against the redox pair of ferrocene/ferrocenium, reaching the oxidation peak of *anti*M+*syn*M (anodic mode),
- sweeping potential between -1.75 V and 1 V against the redox pair ferrocene/ferrocenium, reaching the oxidation and reduction peak of *anti*M+*syn*M (anodic-cathodic mode),
- speed rate of the potential: 10 mV/s,
- material of the working electrode - platinum,
- deposition from the solution of *anti*M+*syn*M in toluene with tetrabutylammonium tetrafluoroborate electrolyte, which is dissolved in this solvent,
- deposition from solid state of *anti*M+*syn*M, in dichloromethane and tetrabutylammonium tetrafluoroborate electrolyte, which is dissolved in this solvent,
- time of the process - up to the precipitation of the PPPI product on the working electrode.

### Example III

Example III differs from the I in that the PPPI is obtained using the following procedure.

A mixture of *anti*M+*syn*M monomers (*anti* perimidino [1',2':1,5]pyrrolo[3,4-*m*]phthaloperine-9,19-dione and *syn* 17H,19H-perimidino[1',2':1,2]pyrrolo[3,4-*m*]phthaloperine-17,19-dione, preferably in a molar ratio of 0:1, is subjected to potentiodynamic polymerization (cyclic voltammetry) under the conditions:
sweeping potential between 0.5 V and 1 V against the redox pair of ferrocene/ferrocenium, reaching the oxidation peak of *anti*M+*syn*M (anodic mode),
- sweeping potential between -1.75 V and 1 V against the redox pair ferrocene/ferrocenium, reaching the oxidation and reduction peak of *anti*M+*syn*M (anodic-cathodic mode),,
- speed rate of the potential: 500 mV/s,
- material of the working electrode - platinum alloys,
- deposition from the solution of *anti*M+*syn*M in benzene with tetrabutylammonium hexafluorophosphate electrolyte, which is dissolved in this solvent,
- deposition from solid state of *anti*M+*syn*M, in chlorobenzene with tetrabutylammonium hexafluorophosphate electrolyte, which is dissolved in this solvent,
- time of the process - up to the precipitation of the PPPI product on the working electrode.

### Example IV

Example IV differs from the I in that the PPPI is obtained using the following procedure.

A mixture of *anti*M+*syn*M monomers (*anti* perimidino [1',2':1,5]pyrrolo[3,4-*m*]phthaloperine-9,19-dione and *syn* 17H,19H-perimidino[1',2':1,2]pyrrolo[3,4-*m*]phthaloperine-17,19-dione, preferably in a molar ratio of 0.5:0.5, is subjected to potentiodynamic polymerization (cyclic voltammetry) under the conditions:
- sweeping potential between 0.5 V and 1 V against the redox pair of ferrocene/ferrocenium, reaching the oxidation peak of *anti*M+*syn*M (anodic mode),
- sweeping potential between -1.75 V and 1 V against the redox pair ferrocene/ferrocenium, reaching the oxidation and reduction peak of *anti*M+*syn*M (anodic-cathodic mode),
- speed rate of the potential: 250 mV/s,
- material of the working electrode - glassy carbon,
- deposition from the solution of *anti*M+*syn*M in toluene with tetraethylammonium hexafluorophosphate electrolyte, which is dissolved in this solvent,
- deposition from solid state of *anti*M+*syn*M, in 1,2-chlorobenzene with tetraethylammonium hexafluorophosphate, tetrabutylammonium hexafluorophosphate or tetraethylammonium hexafluorophosphate electrolyte, which is dissolved in this solvent,
- time of the process - up to the precipitation of the PPPI product on the working electrode.

## Claims

1. A new perinone polymer with the general formula containing units marked as u, w, v, x, y, z: 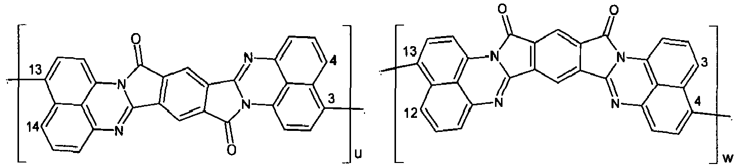 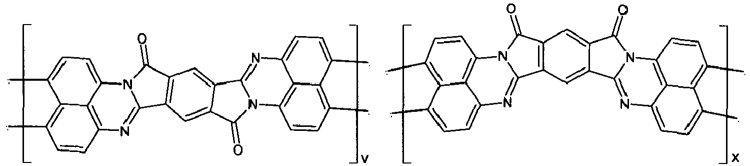 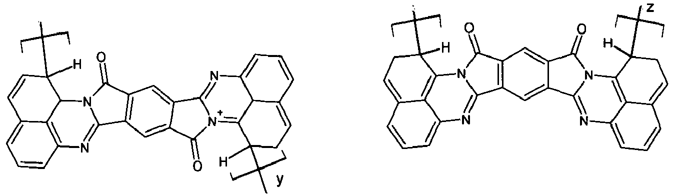 where:
u - poly(*anti*M) by single bond via positions 1, 2, 3, 4, 5, 6, 11, 12, 13, 14, 15 and/or 16
w - poly(*syn*M) by single bond via positions 1, 2, 3, 4, 5, 6, 10, 11, 12, 13, 14 and/or 15
v - poly(*anti*M) by double bond via positions 1, 2, 3, 4, 5, 6, 11, 12, 13, 14, 15 and/or 16
x - poly(*syn*M) by double bond via positions 1, 2, 3, 4, 5, 6, 10, 11, 12, 13, and/or 15
y - poly(*anti*M) by unprotonated bond via positions 1, 2, 3, 4, 5, 6, 11, 12, 13, 14, 15 and/or 16
z - poly(*syn*M) by unprotonated bond via positions 1, 2, 3, 4, 5, 6, 10, 11, 12, 13, 14 and/or 15

2. Method for obtaining a perinone polymer by potentiodynamic polymerization, which is characterized as the mixture of *anti*M+*syn*M monomers *(anti* perimidino[1',2':1,5]pyrrolo[3,4-*m*]phthaloperine-9,19-dione an*d syn* 17H,19H-perimidino[1',2':1,2]pyrrolo[3,*4*-*m*] phthaloperine-17,19-dione, preferably in a molar ratio in the range 1:0 to 0:1, is subjected to potentiodynamic polymerization (cyclic voltammetry) on a working electrode made of metallic or carbon materials; preferably platinum, a platinum alloy or a glassy carbon, where the process is carried out under the following conditions:
- sweeping potential between 0.5 V and 1 V against the redox pair of ferrocene/ferrocenium, reaching the oxidation peak of *anti*M+*syn*M (anodic mode),
- sweeping potential between -1.75 V and 1 V against the redox pair ferrocene/ferrocenium, reaching the oxidation and reduction peak of *anti*M+*syn*M (anodic-cathodic mode),
- speed rate of the potential in the range of 10 - 500 mV/s,
- deposition from the solution of *anti*M+*syn*M, solvents, preferably toluene, benzene, and an electrolyte that is dissolved in a solvent, preferably tetrabutylammonium tetrafluoroborate, tetrabutylammonium hexafluorophosphate or tetraethylammonium hexafluorophosphate,
- deposition from solid state of *anti*M+*syn*M, solvents, e.g. dichloromethane, chlorobenzene, 1,2-dichlorobenzene, methanol and electrolyte which is dissolved in a solvent, e.g. tetrabutylammonium tetrafluoroborate, tetrabutylammonium hexafluorophosphate, tetraethylammonium hexafluorophosphate,
- the process is carried out until the PPPI product is precipitated on the working electrode.
